Europäisches Patentamt

(19) European Patent Office

Office européen des brevets

(11) Publication number: **0 067 467**
**A1**

(12) **EUROPEAN PATENT APPLICATION**

(21) Application number: 82200637.5

(22) Date of filing: 25.05.82

(51) Int. Cl.³: **C 07 C 5/25**
**C 07 C 11/02, B 01 J 27/02**

(30) Priority: 15.06.81 US 273533

(43) Date of publication of application:
22.12.82 Bulletin 82/51

(84) Designated Contracting States:
DE FR GB NL

(71) Applicant: SHELL INTERNATIONALE RESEARCH
MAATSCHAPPIJ B.V.
Carel van Bylandtlaan 30
NL-2596 HR Den Haag(NL)

(72) Inventor: Ryan, Robert Charles
4914 Gleneagles
Houston Texas 77084(US)

(72) Inventor: Slaugh, Lynn Henry
610 Winter Oaks
Houston Texas 77079(US)

(74) Representative: Puister, Antonius Tonnis, Mr. et al,
P.O. Box 302
NL-2501 CH The Hague(NL)

(54) Process for the double-bond isomerization of olefins.

(57) A process for the double-bond isomerization of olefins with minimal skeletal isomerization in which one or more olefins are contacted under isomerization conditions with a catalyst comprising partially hydrated chromium sulphate on a porous inert siliceous support. The catalyst is prepared by impregnating the porous support with an aqueous chromium sulphate solution, drying, and activating at a temperature ranging from 120°C to 240°C.

EP 0 067 467 A1

Croydon Printing Company Ltd.

- 1 -

## PROCESS FOR THE DOUBLE-BOND ISOMERIZATION OF OLEFINS

This invention relates to a process for the double-bond isomerization of olefins utilizing a chromium sulphate supported catalyst.

Isomerization of an olefin can comprise either a skeletal rearrangement and/or a double-bond shift. There are commercial processes wherein a double-bond shift of, for example, an alpha-olefine provides a very useful feedstock for other processes such as, for example, an alkylation process or a disproportionation process.

U.S. patent specification 3,301,916 discloses the use of an anhydrous chromium sulphate supported catalyst for isomerizing olefins containing for example 4-6 carbon atoms. The isomerization comprises both double-bond shift and skeletal rearrangement.

It has now been found that catalysts comprising a partially hydrated chromium sulphate supported on a porous inert siliceous support are highly selective towards double-bond shift isomerization and cause only minimal skeletal rearrangement.

According to the invention there is provided a process for the double-bond isomeration of olefins in which one or more olefins are contacted under isomerization conditions with a catalyst comprising chromium sulphate on a porous inert siliceous support, characterized in that the catalyst contains a partially hydrated chromium sulphate.

Suitable porous inert siliceous supports are those typically utilized as catalyst supports in the industry. Preferred supports are for example silica and kieselguhr.

The porous silicas which may be used to prepare the catalyst are readily available commercially and are known as silica gels which are essentially substantially dehydrated amorphous silica. These materials are available in various density grades, from

- 2 -

low density with surface areas ranging from 100-200 $m^2/g$, to regular density with surface areas up to 800 $m^2/g$. These commercially available materials are used as desiccants, selective adsorbents, catalysts and catalyst supports. The porous silica may contain minor proportions of other materials such as for example alumina and carbon. Kieselguhr and diatomaceous earths are readily available commercially and make excellent supports for the catalysts used according to the invention. Examples of commercially available silica gels and their properties are shown in the table below.

| Support | Surface area, $m^2/g$ | Pore vol., $ml/g$ | Density $g/ml$ | Particle size |
|---|---|---|---|---|
| Davison* Grade 952 $SiO_2$ | 300 | 1.65 | 0.35 | 70 mesh (avg.) (0.21 mm) |
| Davison Grade 57 $SiO_2$ | 300 | 1.0 | 0.4 | < 100 mesh (< 0.149 mm) |
| Davison Grade 03 $SiO_2$ | 750 | 0.43 | 0.7 | 8 mesh (avg.) (2.38 mm) |

*Manufactured by Davison Chemical Div., W.R. Grace & Co.

To prepare the catalyst used in the process according to the invention, the porous support is impregnated with an aqueous solution of chromium sulphate, dried, for example, at temperatures up to 120°C, and subsequently heated to a temperature ranging from 120°C to 240°C, preferably from 140°C to 190°C. The drying and heating step may be carried out in one single sequence through a programmed temperature drying process. This final heating step will leave a molar ratio of $H_2O/Cr$ (metal) in the catalyst ranging from 0.7 to 1.1, preferably from 0.8 to 1.0. This final heating step is critical. Heating the material to higher temperatures say 350°C and higher substantially dehydrates the chromium sulphate. The impregnation may be a single step impregnation, preferably a so-called "dry impregnation" wherein only that amount of aqueous solution is utilized which just fills the voids in the catalyst support. Alternatively, the catalyst may be impregnated several times to provide different levels of chromium sulphate. The final catalyst will contain 0.1 to 25, preferably 1 to 10 percent by weight of chromium measured as the metal.

The isomerization process is carried out by contacting the catalyst with the appropriate olefin or mixture of olefins under isomerization conditions. The exact conditions will depend upon the particular olefin or olefins being isomerized and these conditions can readily be determined by routine experimentation. In general, isomerization conditions would encompass temperatures in the range of 50°C to 500°C, preferably from 50°C to 350°C, and most preferably from 50°C to 200°C, and pressures ranging from sub-atmospheric to super-atmospheric, for example ranging from 0.1 atmosphere to 100 atmospheres, although other pressures can readily be utilized. The isomerization can be performed as either a batch, continuous or a semi-continuous process. A fixed, moving or fluidized catalyst bed may be employed; and the contacting of the catalyst with the hydrocarbons may performed either in the presence of or the absence from a diluent that is substantially inert with respect to the catalyst under conditions employed.

The feedstock to the process may be any olefin capable of isomerization, preferred olefins are, however, alpha-olefins. These may be either lower molecular weight olefins ranging from for example, $C_4$ to $C_6$, or the higher range olefins ranging from for example $C_6$ to $C_{50}$. Preferably, the process is carried out with a hydrocarbon liquid hourly space velocity in the range of 0.1 to 10 when utilizing a fixed bed configuration.

- 4 -

The process of the invention will be further described below the the following Examples.

EXAMPLE 1

Catalyst preparation

A porous silica gel (Davison Grade 57, surface area 300 $m^2/g$, pore volume 1.0 ml/g, density 0.4 g/ml, and particle size 20-30 mesh (0.59-0.84 mm)) is pretrated in dry nitrogen at 600°C for 2 hours. To 12 grams of this dried material is added a solution of 4.12 grams of $Cr_2(SO_4)_3 \cdot 12H_2O$ dissolved in 15 millilitres of water. After impregnation the material is dried with a hot air gun and then further dried at 125°C for 1 hour in a muffled furnace. The catalyst subsequently activated at the appropriate temperature in situ in the olefin isomerization reactor.

Isomerization process

The procedure for catalyst testing consists of mixing 2.5 grams of the catalyst as prepared above with 2.5 grams of Davison 57 $SiO_2$ and placing this in a glass reactor tube (15 mm diameter). The remaining space in the reactor tube above the catalyst bed is filled with Davison 57 $SiO_2$ (approximately 5 grams). The tube is placed in a furnace, and the catalyst is activated by heating it under a stream of nitrogen where it passes down through the bed. The olefin is pumped in a down flow mode through the bed at a predetermined temperature. With the use of higher molecular weight olefins, parts of the pumping system are heated to approximately 70°C in order to prevent plugging of the lines. During all operations a slow nitrogen or argon flow is maintained over the feed and throughout the flow system.

Catalysts are also prepared using Kaiser KA201 alumina (surface area 365 $m^2/g$, pore volume 0.42 ml/g (comparative experiment), and Davison silica/alumina (grade 980-25, 75% silica-25% alumina, surface area 325 $m^2/g$, pore volume 0.45 ml/g). The catalysts are prepared to have a final weight of chromium sulphate of 2.1% basis chromium metal. Silica supported catalysts are activated at either 150°C or 350°C. At 150°C the catalysts contain approximately 1.9% weight of water ($H_2O/Cr$ ratio of approximately 0.9). The catalyst activated at 350°C contains substantially no water.

The isomerization reactor is heated to the reaction temperature specified in Table 1 and hexene is pumped into the reactor at the weight hour space velocity given in Table 1. The products are analysed by gas chromatography and the results are shown in Table 1. As can be seen the catalyst that is activated at 150°C is far more active than the substantially anhydrous catalyst activated at 350°C. The former catalyst even when operated at twice the weight hourly space velocity of hexene provides much more of the isomerized product than does the substantially anhydrous catalyst. The chromium sulphate supported on alumina provides substantially no isomerization under the conditions of this example.

TABLE 1

## SUPPORTED CHROMIUM SULPHATE ISOMERIZATION CATALYSTS

### 1-HEXENE ISOMERIZATION

#### Activation Conditions

| Example | Support | Cr (wt%) | Temp. (°C) | Gas | Time (hr) | Reaction temp. (°C) | WHSV | Hexene products 1- | 2- & 3- | Dimers |
|---------|---------|----------|------------|-----|-----------|---------------------|------|------|---------|--------|
| 1-1 | SiO$_2$ (Gr57) | 2.1 | 150 | N$_2$ | 1.0 | 85 | 10.0 | 5.1 | 93.9 | 1.0 |
| 1-2 | SiO$_2$ (Gr57) | 2.1 | 350 | N$_2$ | 3.0 | 93 | 5.0 | 11.6 | 87.2 | 1.2 |
| 1-3 | Al$_2$O$_3$ (KA201) | 2.1 | 150 | N$_2$ | 2.0 | 120 | 5.0 | 100.0 | 0.0 | 0.0 |
| 1-4 | SiO$_2$/Al$_2$O$_3$ (980-25) | 2.1 | 150 | N$_2$ | 3.0 | 112 | 5.0 | 13.4 | 85.5 | 1.1 |

EXAMPLE 2

By means of the method described in Example 1, samples are prepared containing 4.2% wt chromium (basis chromium metal). These catalysts are placed in the reactor and activated both in nitrogen and in air. The results are shown in Table 2.

EXAMPLE 2

## TABLE 2

### SUPPORTED CHROMIUM SULPHATE ISOMERIZATION CATALYSTS
### 1-HEXENE ISOMERIZATION

#### Activation Conditions

| Example | Support | Cr (wt%) | Temp. (°C) | Gas | Time (hr) | Reaction temp. (°C) | WHSV | Hexene products | | |
|---------|---------|----------|------------|-----|-----------|---------------------|------|------|-----|------|
| | | | | | | | | 1- | 2- & 3- | Dimers |
| 2-1 | $SiO_2$ (Gr57) | 4.2 | 150 | $N_2$ | 3.0 | 78 | 7.0 | 9.8 | 89.6 | 0.6 |
| 2-2 | $SiO_2$ (Gr57) | 4.2 | 150 | air | 3.0 | 81 | 7.0 | 2.4 | 95.9 | 1.7 |

EXAMPLE 3

By means of the method described in Example 1 a series of catalysts containing various amounts of chromium are prepared. These are activated in nitrogen at 150°C for 3 hours and subsequently 175°C for 1 hour. As a feed, a mixture of higher molecular weight olefins is utilized which results from a process which comprises oligomerizing ethylene to olefins, and then subsequently isomerizing and disproportionating the olefins to produce an olefin mixture containing $C_6$ to $C_{50}$ olefins and having an average carbon number of 15.4. The reaction is carried out at various temperatures indicated in Table 3 and the results of these experiments are also shown in Table 3.

## TABLE 3

ISOMERIZATION OF HIGH MOLECULAR WEIGHT OLEFINS[a]
WITH $Cr_2(SO_4)_3/SiO_2$ CATALYSTS[b]

| Example | Cr (wt%) | Temp. (°C) | WHSV[c] | %[d] |
|---|---|---|---|---|
| Starting Material | - | - | - | - |
| 3-1 | 2.1 | 110 | 4.4 | 84 |
| 3-2 | 2.1 | 133 | 4.4 | 89 |
| 3-3 | 2.1 | 130 | 2.6 | 100 |
| 3-4 | 4.2 | 102 | 4.7 | 75 |
| 3-5 | 4.2 | 115 | 4.7 | 95 |
| 3-6 | 4.2 | 130 | 4.7 | 87 |
| 3-7 | 4.2 | 133 | 9.0 | 73 |
| 3-8 | 4.2 | 129 | 2.2 | 100 |
| 3-9 | 4.2 | 133 | 21.6 | 67 |
| 3-10 | 5.2 | 98 | 4.5 | 85 |
| 3-11 | 5.2 | 120 | 4.7 | 74 |
| 3-12 | 5.2 | 133 | 4.6 | 90 |
| 3-13 | 5.2 | 135 | 9.1 | 62 |
| 3-14[e,f] | 5.2 | 102 | 4.7 | 100 |
| 3-15 | 5.2 | 121 | 4.7 | 100 |

[a] A full range ($C_6$ to $C_{50}$) olefins are used with an average carbon number of 15.4.

[b] The $Cr_2(SO_4)_3/SiO_2$ catalysts are heated under $N_2$ at 150°C for 3 hours and 175°C for 1 hour.

[c] WHSV = weight hourly space velocity.

[d] % isomerization towards equilibrium.

[e] Reaction is performed with upflow of olefin substate.

[f] Catalyst is activated under $N_2$ at 125°C for 3 hrs. Olefins are purified over KA-201 $Al_2O_3$ at 100°C prior to isomerization.

- 11 -

To determine skeletal isomerization, samples of the above isomerized olefins are analyzed by $^{13}$C NMR. The basis of this analysis is to use the ratio of integrals from methyl group carbons ($I_c(CH_3)$) and also those from the total carbon atoms ($I_c$(total) and to relate these to the percentage of molecules with branches. An increase in this ratio with isomerization would indicate an increase in skeletal isomerization. The results are shown in Table 4. It can be seen from this table that no significant skeletal isomerization occurs.

## TABLE 4

ANALYSIS OF BRANCHING IN ISOMERIZED HIGH MOLECULAR
WEIGHT OLEFINS BY NMR[a]

| Sample | Average $N^{[b]}$ | $\dfrac{I_c(CH_3)^{[c]}}{I_c(total)}$ | Isomerization temp.(°C) | % Iso-merization |
|---|---|---|---|---|
| Starting Materials | 15.4 | 0.14 | – | 0 |
| 4-1 | 15.4 | 0.16 | 133 | 89 |
| 4-2 | 15.4 | 0.16 | 130 | 100 |
| 4-3 | 15.4 | 0.14 | 102 | 75 |
| 4-4 | 15.4 | 0.15 | 115 | 95 |

[a] All samples are isomerized at the indicated temperature with a $Cr_2(SO_4)_3/SiO_2$ catalyst (4.1 wt% Cr) and hydrogenated with 10% pt/C at 250 psi(17.24 bar)$H_2$ and 70°C.

[b] Average carbon number (N) is determined by G.C. analysis.

[c] $I_c(CH_3)$ and $I_c$ (total) are the methyl and total carbon integrals, respectively.

## C L A I M S

1. A process for the double-bond isomerization of olefins in which one or more olefins are contacted under isomerization conditions with a catalyst comprising chromium sulphate on a porous inert siliceous support, characterized in that the catalyst contains a partially hydrated chromium sulphate.

2. A process as claimed in claim 1, characterized in that the support is silica or kieselguhr.

3. A process as claimed in claim 1 or 2, characterized in that the amount of chromium present in the catalyst ranges from 0.1 to 25 percent by weight based on chromium metal.

4. A process as claimed in claim 3, characterized in that the amount of chromium present in the catalyst ranges from 1 to 20 percent by weight based on chromium metal.

5. A process as claimed in any one of the claims 1 or 4, characterized in that the molar ratio of water to chromium metal in the catalyst ranges from 0.7 to 1.1.

6. A process as claimed in claim 5, characterized in that the molar ratio of water to chromium metal in the catalyst ranges from 0.8 to 1.0.

7. A process as claimed in any one of the claims 1 or 6, characterized in that the catalyst is prepared by impregnating the porous inert siliceous support with an aqueous solution of chromium sulphate, drying the impregnated support, and activating the dried support at a temperature ranging from 120°C to 240°C.

8. A process as claimed in claim 7, characterized in that the dried support is activated at a temperature ranging from 140°C to 190°C.

9. A process as claimed in any one of the claims 1 or 8, characterized in that the isomerization is carried out at a temperature ranging from 50°C to 200°C.

10. A process as claimed in any one of the claims 1 or 9, characterized in that the isomerization is carried out at a temperature ranging from 0.1 to 100 atm.

**0067467**

European Patent
Office

EUROPEAN SEARCH REPORT

Application number

EP 82 20 0637.5

| DOCUMENTS CONSIDERED TO BE RELEVANT | | | |
|---|---|---|---|
| Category | Citation of document with indication, where appropriate, of relevant passages | Relevant to claim | |
| X | <u>GB - A - 1 003 003</u> (BP)<br>* claims 1, 2; example 3 *<br>-- | 1,7-9 | |
| A,D | <u>US - A - 3 301 916</u> (R.C. PITKETHLY et al.)<br>* claims 1, 4; example 5 *<br>-- | 1,2 | |
| A | <u>DE - B - 1 185 169</u> (SICEDISON)<br>* claim 1; column 3, lines 53 to 62 *<br>----- | 1 | |

**CLASSIFICATION OF THE APPLICATION (Int. Cl. 3)**

C 07 C  5/25

C 07 C 11/02

B 01 J 27/02

**TECHNICAL FIELDS SEARCHED (Int.Cl. 3)**

B 01 J 23/26

B 01 J 27/02

C 07 C  5/23

C 07 C  5/25

C 07 C 11/02

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| Berlin | 30-08-1982 | KNAACK |

EPO Form 1503.1   06.78